(19) Europäisches Patentamt
European Patent Office
Office européen des brevets

(11) **EP 2 520 181 A1**

(12) **EUROPEAN PATENT APPLICATION**

(43) Date of publication:
**07.11.2012 Bulletin 2012/45**

(51) Int Cl.:
*A23L 1/30* (2006.01)          *A61P 1/00* (2006.01)
*A23L 1/29* (2006.01)          *A23L 1/305* (2006.01)
*A61K 35/74* (2006.01)

(21) Application number: **11164461.3**

(22) Date of filing: **02.05.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **N.V. Nutricia**
**2712 HM  Zoetermeer (NL)**

(72) Inventors:
• **Ludwig, Thomas**
**6704 PH Wageningen (NL)**

• **Huybers, Sylvie**
**6581 RG Malden (NL)**
• **Abrahamse, Evan**
**3815 ST Amersfoort (NL)**
• **Bouritius, Houkje**
**3705 SC Zeist (NL)**

(74) Representative: **Swinkels, Bart Willem**
**Nederlandsch Octrooibureau**
**J. W. Frisolaan 13**
**2517 JS Den Haag (NL)**

(54) **Fermented infant formula**

(57)    The present invention relates to a fermented infant formulae for decreasing protein digestive effort by decreasing the amount of endogenously formed proteases, concomitant with an increased protein digestion. The invention also relates to low protein fermented infant formulae.

EP 2 520 181 A1

**Description**

FIELD

The present invention relates to nutritional formulae for infants and toddlers and in particular addresses protein digestion.

BACKGROUND

Digestion of dietary proteins is achieved by the release of proteases from the stomach and the pancreas. The release of proteases is normally tightly regulated, thereby ensuring that not too less and not too much proteolytic enzymes are secreted. This is important since a high release of proteases and a high proteolytic activity has several disadvantages. A too high release of proteases will result in energy loss and a loss of essential amino acids due to the fact that proteases themselves are highly resistant to proteolytic activity and will enter into the colon mostly unaltered. Subsequently, upon entering of an increased amount of protein (in the form of proteases) into the colon the intestinal microbiota will ferment the proteins resulting in a disadvantageously higher pH, a shift in the composition of the intestinal microbiota and formation of toxic metabolites such as phenol, indol, and amines. This change in colonic physiology may also lead to increased intestinal infections. Finally, proteases such as trypsin are known to cleave protease activated receptors (PARs), such as PAR-II, and thereby disrupt intestinal barrier integrity. This may result in an increased abdominal pain perception Inflammatory diseases such as IBS-D and UC have been linked to elevated levels of proteolytic activity within the intestinal lumen and subsequent PAR-II activation. Furthermore, increased faecal proteolytic activity is linked to diaper rashes. In general, a too high release of proteases will result in decreasing gastrointestinal comfort, functional digestive disorders, gastrointestinal gas formation, and/or bloating.
A too low secretion of proteolytic activity, on the other hand, is disadvantageous since in that case the dietary proteins are not properly digested leading also to loss of essential amino acids and energy, and an increased protein load of the colon.
Especially in infants a tight regulation of protein digestion and release of proteolytic activity is of utmost importance. Firstly, for infants a limited loss of protein is essential for good growth and development. Loss of essential amino acids and energy impairs growth and development. Secondly, the intestinal barrier function in infants is still immature and the intestinal microbiota is still developing and therefore more susceptible for the disadvantages mentioned above.
Known ways to improve protein digestion, in particular in infants, involve partial predigestion of dietary proteins by proteases. Furthermore, Alm, 1982, J Dairy Sci 65:509-514 discloses that a low pH of milk products, especially as a result of fermentation, has a positive influence on *in vitro* digestibility of proteins. It is considered that in many digestive disorders the secretion of hydrochloric acid is impaired and thus the suitability of such low pH milk products is suggested for infants, children and adults. Vass et al, 1984, Acta Medica Hungarica, 41, 15-161 disclose that fermented milks have the highest protein utilization index, defined as increase in body mass in g per protein intake in g, in weaning rats, and this is attributed to a better digestibility of proteins.
Gallia Lactofidus® is an acidified infant formula, resulting from fermentation by two specific strains of lactic acid bacteria. It is disclosed that this formula facilitates the digestibility of proteins and improves the intestinal transit.

SUMMARY OF THE INVENTION

The inventors have found that, employing an animal model with piglets, upon consumption of a fermented formula the amount of endogenous proteolytic enzymes that was detected at the terminal ileum was significantly reduced compared to the amount detected upon consumption of a standard formula. Surprisingly, the amount was also significantly reduced compared to the amount detected upon consumption of a formula with extensively hydrolysed (i.e. predigested) proteins. In addition and unexpectedly as well, even though the daily protein intake was higher in the piglets consuming the fermented formula the apparent and true protein digestibility was the highest in this group as well. This is indicative for a decreased digestive effort. Digestive effort is defined as the amount of protease activity secreted per gram of ingested protein. Digestive efficacy is defined as reciprocal value of digestive effort. This means that at high digestive efficacy (little protease needed to digest the ingested protein) the digestive effort is low. Strikingly, it was found that the proteolytic activity in faecal samples of exclusively breast fed human infants was lower than that of infants fed with a standard formula. Therefore the use of a fermented formula advantageously is likely to make the protease activity in the colon more reminiscent to the situation as that in breast fed infants. Upon feeding a fermented formula the release of endogenous protease, protein digestion efficacy, digestive effort, endogenous protein loss and the protein load entering the colon is improved. It becomes more similar to the situation in breast fed infants than when feeding a standard non fermented infant formula. Therefore a fermented formula is advantageously used to feed infants for use in preventing and/or treatment of diaper rashes or in promoting gut health by reducing digestive effort, improving protein digestion efficacy, reducing endogenous protein loss, reducing endogenous secretion of proteases, and/or reducing the protein load entering the colon.

The finding that release of endogenous protease is reduced, protein digestion efficacy is increased, and endogenous protein loss is decreased also enables the formulation of an infant formula with lower protein concentrations than used sofar. Therefore the present invention also relates to fermented infant formula with low protein concentration.

DETAILED DESCRIPTION

The present invention concerns a method, in particular a non-therapeutic method, for

a reducing digestive effort,
b improving protein digestion efficacy,
c reducing endogenous protein loss,
d reducing endogenous secretion ofproteases, and/or
e reducing the protein load entering the colon

compared to a nutritional composition not comprising a protein comprising composition fermented by lactic acid bacteria, said method comprising administering a nutritional composition that comprises from 5 to 20 wt.% protein based on dry weight of the nutritional composition and that comprises at least 10 wt.% based on dry weight of the nutritional composition of a protein comprising composition fermented by lactic acid bacteria.

In a preferred embodiment, the present method is for reducing digestive effort.

In one aspect the invention concerns a method for prevention and/or treatment of diaper rashes, said method comprising administering to an infant a nutritional composition that comprises from 5 to 20 wt.% protein based on dry weight of the nutritional composition and that comprises at least 10 wt.% based on dry weight of the nutritional composition of a protein comprising composition fermented by lactic acid bacteria.

In other words the invention concerns the use of a protein comprising composition fermented by lactic acid bacteria in the manufacture of a nutritional composition for prevention and/or treatment of diaper rashes wherein the nutritional composition comprises from 5 to 20 wt.% protein based on dry weight of the nutritional composition and that comprises at least 10 wt.% based on dry weight of the nutritional composition of the protein comprising composition fermented by lactic acid bacteria.

The invention can also be worded as a nutritional composition that comprises from 5 to 20 wt.% protein based on dry weight of the nutritional composition and that comprises at least 10 wt.% based on dry weight of the nutritional composition of a protein comprising composition fermented by lactic acid bacteria, for use in prevention and/or treatment of diaper rashes.

In one aspect the present invention concerns a method for promoting gut health, said method comprising administering a nutritional composition that comprises from 5 to 20 wt.% protein based on dry weight of the nutritional composition and that comprises at least 10 wt.% based on dry weight of the nutritional composition of a protein comprising composition fermented by lactic acid bacteria.

In other words the invention concerns the use of a protein comprising composition fermented by lactic acid bacteria in the manufacture of a nutritional composition for promoting gut health wherein the nutritional composition comprises from 5 to 20 wt.% protein based on dry weight of the nutritional composition and that comprises at least 10 wt.% based on dry weight of the nutritional composition of the protein comprising composition fermented by lactic acid bacteria.

The invention can also be worded as a nutritional composition that comprises from 5 to 20 wt.% protein based on dry weight of the nutritional composition and that comprises at least 10 wt.% based on dry weight of the nutritional composition of a protein comprising composition fermented by lactic acid bacteria, for use in promoting gut health in an infant.

In a further aspect the invention concerns a method for promoting gut health by

a reducing digestive effort,
b improving protein digestion efficacy
c reducing endogenous protein loss
d reducing endogenous secretion of proteases, and/or
e reducing the protein load entering the colon

compared to a nutritional composition not comprising a protein comprising composition fermented by lactic acid bacteria, said method comprising administering a nutritional composition that comprises from 5 to 20 wt.% protein based on dry weight of the nutritional composition and that comprises at least 10 wt.% based on dry weight of the nutritional composition of a protein comprising composition fermented by lactic acid bacteria.

In other words the invention concerns the use of a protein comprising composition fermented by lactic acid bacteria in the manufacture of a nutritional composition for promoting gut health by

    a reducing digestive effort,
    b improving protein digestion efficacy
    c reducing endogenous protein loss
    d reducing endogenous secretion of proteases, and/or
    e reducing the protein load entering the colon

compared to a nutritional composition not comprising a protein comprising composition fermented by lactic acid bacteria, wherein the nutritional composition comprises from 5 to 20 wt.% protein based on dry weight of the nutritional composition and that comprises at least 10 wt.% based on dry weight of the nutritional composition of the protein comprising composition fermented by lactic acid bacteria.

The invention can also be worded as a nutritional composition that comprises from 5 to 20 wt.% protein based on dry weight of the nutritional composition and that comprises at least 10 wt.% based on dry weight of the nutritional composition of a protein comprising composition fermented by lactic acid bacteria, for use in promoting gut health by

    a reducing digestive effort,
    b improving protein digestion efficacy
    c reducing endogenous protein loss
    d reducing endogenous secretion of proteases, and/or
    e reducing the protein load entering the colon

compared to a nutritional composition not comprising the protein comprising composition fermented by lactic acid bacteria.

For sake of clarity it is noted that the "nutritional composition" mentioned above refers to the final nutritional composition that is to be ingested or administered and the "protein comprising composition fermented by lactic acid bacteria" is comprised in the nutritional composition. The nutritional composition thus may also be referred to as "final nutritional composition" or "total nutritional composition". The "protein comprising composition fermented by lactic acid bacteria" may also be referred to as "protein comprising fermented composition" or "fermented ingredient". Also, further for sake of clarity it is noted that the "protein" of the "protein comprising composition fermented by lactic acid bacteria" is comprised in the "5 to 20 wt.% protein" of the nutritional composition.

Further, the invention also concerns a nutritional compostion for infants comprising:

    a. protein

        i. in an amount of from 5 to 20 wt.% based on dry weight of the nutritional composition, and
        ii. in an amount of from 6 to 7.4 % calories based on total calories of the nutritional composition,

    b. lipids in an amount of from 35 to 60 % calories based on total calories of the nutritional composition, and
    c. carbohydrates in an amount of from 40 to 60 % calories based on total calories of the nutritional composition,

wherein the nutritional composition comprises at least 10 wt.% of a protein comprising composition fermented by lactic acid bacteria based on dry weight of the nutritional composition.

The term "nutritional composition" used throughout this description, i.e. the final nutritional composition that is to be ingested or administered, also refers to the "nutritional composition for infants" specified here above.

*Fermented ingredient*

The present nutritional composition comprises a protein comprising composition fermented by lactic acid bacteria. The present nutritional composition preferably comprises a fermented milk-derived protein comprising composition. This fermented milk-derived protein comprising composition is obtained by incubation of a combination of milk, e.g. skim milk, or a milk derived product, e.g. whey, with at least one strain of lactic acid bacterium, such as lactococci, lactobacilli, streptococci and bifidobacteria, preferably with *Streptococcus thermophilus.* Preferably the combination is incubated for 10 minutes to about 6 hours. The temperature during incubation is preferably between 20 and 50 °C. After incubation the incubated composition is preferably subjected to a heat treatment. By this heat treatment preferably at least 90 % of living microorganisms are inactivated. The heat treatment preferably is performed at a temperature between 80 and 180 °C. Inactivation of the lactic acid bacterium advantageously results in less post acidification and a safer product.

This is especially advantageous when the nutritional composition is to be administered to infants. Procedures to prepare fermented ingredients suitable for the purpose of the present invention are known per se. EP 778885, which is incorporated herein by reference, discloses in particular in example 7 a suitable process for preparing a fermented composition. FR 2723960, which is incorporated herein by reference, discloses in particular in example 6 a suitable process for preparing a fermented composition. Briefly, a milk derived product, preferably pasteurised, containing lactose and optionally further macronutrients such as fats, preferably vegetable fats, casein, whey protein, vitamins and/or minerals etc. is concentrated, e.g. to between 15 to 50% dry matter and then inoculated with *S. thermophilus*, for example with 5% of a culture containing $10^6$ to $10^{10}$ bacteria per ml. Preferably this milk derived product comprises milk protein peptides. Temperature and duration of fermentation are as mentioned above. Suitably after fermentation the fermented protein comprising composition may be pasteurised or sterilized and for example spray dried or lyophilised to provide a form suitable to be formulated in the end product.

The bacterial strains of *S. thermophilus* that are preferably used to prepare the fermented protein comprising composition for the purpose of the present invention develop beta-galactosidase activity in the course of fermentation of the substrate. Preferably beta-galactosidase activity develops in parallel with acidity. Selection of a suitable strain of *S. thermophilus* is described in example 2 of EP 778885 and in example 1 of FR 2723960.

Preferred strains of *S. thermophilus* to prepare protein comprising fermented composition, preferably protein comprising fermented milk-derived composition for the purpose of the present invention have been deposited by Compagnie Gervais Danone at the Collection Nationale de Cultures de Microorganismes (CNCM) run by the Institut Pasteur, 25 rue du Docteur Roux, Paris, France on 23 August 1995 under the accession number I-1620 and on 25 August 1994 under the accession number I-1470.

Preferably, in the preparation of the protein comprising fermented composition additionally other strains of lactic acid bacteria are present or, either simultaneously or consecutively, the composition additionally is fermented by other strains of lactic acid bacteria. Other strains of lactic acid bacteria are preferably selected from the group consisting of Lactobacillus and Bifidobacteria, more preferably *Bifidobacterium breve*, most preferably *Bifidobacterium breve* strain deposited by Compagnie Gervais Danone at the CNCM under number I-2219 on 31 May, 1999.

In one embodiment, the protein comprising composition fermented by lactic acid bacteria, is fermented by *Streptococcus thermophilus,* and/or *Bifidobacterium breve.*

In one embodiment, the nutritional compositon comprises protein comprising composition fermented by lactic acid bacteria wherein the lactic acid bacteria are inactivated after fermentation.

Preferably the present protein comprising fermented composition is not fermented by *Lactobacillus bulgaricus. L. bulgaricus* fermented products are considered not suitable for infants, since in young infants the specific dehydrogenase that converts D-lactate to pyruvate is far less active than the dehydrogenase which converts L-lactate.

The protein comprising fermented composition comprises protein. The protein is preferably selected from the group consisting of non-human animal proteins, preferably milk proteins. The protein comprising fermented composition preferably contains casein, and/or whey protein, more preferably bovine whey proteins and/or bovine casein. The protein comprising fermented composition preferably comprises casein and whey proteins in a weight ratio casein:whey protein of 10:90 to 90:10, more preferably 20:80 to 80:20, even more preferably 35:65 to 55:45.

The protein comprising fermented composition comprises protein preferably providing 5 to 15 % of the total calories of the protein comprising fermented composition, preferably providing 6 to 12%, even more preferable 7 to 8.5 % of the total calories. In one embodiment, the nutritional compositon for infants comprises protein comprising fermented composition wherein the protein preferably provides 5 to 7.5 % of the total calories of the protein comprising fermented composition, preferably provides 6 to 7.4 %, even more preferably 6.5 to 7.3 % of the total calories. When in liquid form, the protein comprising fermented composition preferably comprises 0.5 to 6.0 g, more preferably 1.0 to 3.0 g, even more preferably 1.0 to 1.5 g protein per 100 ml, most preferably 1.0 to 1.3 g protein per 100 ml. Based on dry weight the present protein comprising fermented composition preferably comprises 5 to 20 wt.% protein, preferably at least 8 wt. %, more preferably 8 to 14 wt.%, protein even more preferably 8 to 9.5 wt.% based on dry weight of the protein comprising fermented composition.

The present nutritional composition preferably comprises 10 to 100 wt.% of the protein comprising composition fermented by lactic acid bacteria, preferably a protein comprising fermented milk-derived composition, based on dry weight of the total nutritional composition. In one embodiment the present nutritional composition preferably contains 15 to 70 wt.%, preferably 15 to 50 wt.%, based on dry weight of the final nutritional composition of the protein comprising composition fermented by lactic acid bacteria. Higher concentrations of protein comprising fermented composition advantageously improve the protein digestion efficacy.

The pH of the present nutritional composition is preferably between 5.0 and 8.0, more preferably between 5.0 and 7.5, even more preferably between 5.0 and 6.0, most preferably between 5.5 and 6.0. Preferably the present nutritional composition is a liquid having a pH from 5.5 to 6.0. The present nutritional composition preferably comprises lactic acid and/or lactate. Lactic acid and/or lactate is formed upon fermentation by lactic acid bacteria. Preferably the present nutritional composition comprises between 0.1 and 1.5 wt.% lactic acid and/or lactate, more preferably between 0.2 and 1.0 wt.%, based on dry weight of the nutritional composition. The more lactate is present the more the nutritional composition comprises of the protein comprising fermented composition. Preferably at least 50 wt. %, even more preferably at least 90 wt.%, of the sum of lactic acid and lactate is in the form of L-isomer. Thus in one embodiment the sum of L-lactic acid and L-lactate is more than 50 wt. %, more preferably more than 90 wt.%, based on the sum of total lactic acid and lactate.

In one embodiment the nutritional compostion for infants comprises from 0.10 to 1.5 wt.% of the sum of lactate and lactic acid based on dry weight of the nutritional composition and wherein the sum of L-lactic acid and L-lactate is more than 50 wt. % based on the sum of total lactic acid and lactate.

*Protein component*

The present nutritional composition comprises a protein component. The protein used in the nutritional composition is preferably selected from the group consisting of non-human animal proteins, preferably milk proteins, vegetable proteins, such as preferably soy protein and/or rice protein, free amino acids and mixtures thereof. The present nutritional composition preferably contains casein, and/or whey protein, more preferably bovine whey proteins and/or bovine casein. Thus in one embodiment the protein component comprises protein selected from the group consisting of whey protein and casein, preferably whey protein and casein, preferably the whey protein and/or casein is from cow's milk. The present nutritional composition preferably comprises casein and whey proteins in a weight ratio casein:whey protein of 10:90 to 90:10, more preferably 20:80 to 80:20, even more preferably 35:65 to 55:45.

The wt.% protein based on dry weight of the present nutritional composition is calculated according to the Kjeldah1-method by measuring total nitrogen and using a conversion factor of 6.38 in case of casein, or a conversion factor of 6.25 for other proteins than casein.

The present nutritional composition comprises protein preferably providing 5 to 15 % of the total calories of the nutritional composition, preferably providing 6 to 12%, even more preferable 7 to 8.5 % of the total calories of the nutritional composition. In one embodiment, the nutritional compositon for infants according to the invention comprises protein prefeably providing 5 to 7.5 % of the total calories of the nutritional composition, preferably providing 6 to 7.4 %, even more preferably 6.5 to 7.3 % of the total calories of the nutritional composition. When in liquid form, e.g. as a ready-to-feed liquid, the nutritional composition preferably comprises 0.5 to 6.0 g, more preferably 1.0 to 3.0 g, even more preferably 1.0 to 1.5 g protein per 100 ml, most preferably 1.0 to 1.3 g protein per 100 ml. Based on dry weight the present nutritional composition preferably comprises 5 to 20 wt.% protein, preferably at least 8 wt.% protein based on dry weight of the total nutritional composition, more preferably 8 to 14 wt.%, even more preferably 8 to 9.5 wt.% protein based on dry weight of the total nutritional composition. Since the use of the present nutritional composition results in an increased protein digestion efficacy and a reduced protein digestive effort, the amount of protein based on total calories, based on 100 ml or based on dry weight of the composition advantageously can be lower than that of standard infant formula. The term 'protein' or 'protein component' as used in the present invention refers to the sum of proteins, peptides and free amino acids.

*Nutritional composition*

The present nutritional composition is preferably particularly suitable for providing the complete daily nutritional requirements to a human subject with an age below 36 months, more preferably a human infant. The present nutritional composition is not a yogurt, since yoghurt contains by convention *L. bulgaricus* (Codex Standard for fermented Milks Codex Stan 243-2003).

The present nutritional composition preferably comprises a digestible carbohydrate component. Preferred digestible carbohydrate components are lactose, glucose, sucrose, fructose, galactose, maltose, starch and maltodextrin. Lactose is the main digestible carbohydrate present in human milk. The present nutritional composition preferably comprises lactose. The present nutritional composition preferably comprises digestible carbohydrate, wherein at least 35 wt.%, more preferably at least 50 wt.%, more preferably at least 75 wt.%, of the digestible carbohydrate is lactose. Based on dry weight the present nutritional composition preferably comprises at least 25 wt.% lactose, preferably at least 40 wt.%.

When in liquid form, e.g. as a ready-to-feed liquid, the nutritional composition preferably comprises 3.0 to 30 g digestible carbohydrate per 100 ml, more preferably 6.0 to 20, even more preferably 7.0 to 10.0 g per 100 ml. Based on dry weight the present nutritional composition preferably comprises 20 to 80 wt.%, more preferably 40 to 65 wt.% digestible carbohydrates. Based on total calories the nutritional composition preferably comprises 30 to 60 % calories derived from digestible carbohydrates, more preferably 40 to 60 %.

The present nutritional composition preferably comprises a lipid component. Preferably the lipid component of the present nutritional composition provides 35 to 60 % of the total calories of the nutritional composition, preferably the lipid component provides 40 to 50% of the total calories. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt.% lipid, more preferably 19 to 30 wt.%.

Preferably the lipid component comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition contains at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil and milk fat.

The present nutritional composition is not human breast milk. The present nutritional composition preferably comprises a lipid component and a protein component and a digestible carbohydrate component. The nutritional composition according to the invention or the nutritional composition used according to the invention preferably comprises other fractions, such as vitamins, minerals, trace elements and other micronutrients in order to make it a complete nutritional composition. Preferably the nutritional composition is selected from the group consisting of an infant formula, follow on formula, toddler milk or formula and growing up milk, more preferably form the group consisting of an infant formula and follow on formula. In one embodiment the nutritional composition is an infant formula.. Infant and follow on formulae comprise vitamins, minerals, trace elements and other micronutrients according to international directives.

Preferably the lipid component provides 35 to 60 % of the total calories, the protein component provides 5 to 15% of the total calories and the digestible carbohydrate component provides 30 to 60 %, preferably 35 to 60 % of the total calories of the final nutritional composition. Preferably the present nutritional composition comprises a lipid component providing 40 to 50% of the total calories, a protein component providing 6 to 12% of the total calories and a digestible carbohydrates component providing 40 to 60% of the total calories of the final nutritional composition. The amount of total calories is determined by the sum of calories derived from protein, lipids and digestible carbohydrates.

In one embodiment the nutritional composition is in a liquid form. In another embodiment the nutritional composition is a powder suitable for making a liquid nutritional composition after reconstitution with an aqueous solution, preferably with water. Preferably the infant milk formula is a powder to be reconstituted with water. Preferably the liquid composition has a viscosity below 100 mPa.s, more preferably below 60 mPa.s, more preferably below 35 mPa.s, even more preferably below 6 mPa.s as measured in a Brookfield viscometer at 20°C at a shear rate of 100 s$^{-1}$. A low viscosity is important for infant or follow on formula, since it mimics the viscosity of breast milk and can then be administered via a teat.

In order to meet the caloric requirements of an infant, the nutritional composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/1, more preferably 260 to 320 mOsmol/1. The low osmolarity aims to further reduce the gastrointestinal stress.

When the nutritional composition is in a liquid form, the preferred volume administered on a daily basis is in the range of about 80 to 2500 ml, more preferably about 200 to 1200 ml per day. Preferably, the number of feedings per day is between 1 and 10, preferably between 3 and 8. In one embodiment the nutritional composition is administered daily for a period of at least 2 days, preferably for a period of at least 4 weeks, preferably for a period of at least 8 weeks, more preferably for a period of at least 12 weeks, in a liquid form wherein the total volume administered daily is between 200 ml and 1200 ml and wherein the number of feedings per day is between 1 and 10.

*Application*

The inventors have found that upon consumption of the nutritional composition of the present invention the amount of endogenous proteolytic enzymes that was detected at the terminal ileum was significantly reduced compared to the amount detected upon consumption of a standard, not a fermented ingredient containing nutritional composition. In addition, even though the daily protein intake was higher, the apparent and true protein digestibility was the highest in the group consuming the nutritional composition of the present invention.

The nutritional composition of the present invention was found to reduce digestive effort. Digestive effort is defined as the amount of protease activity secreted per gram of proteins ingested.

The nutritional composition of the present invention was found to improve protein digestion efficacy. Protein digestion efficacy is defined as the amount of protein ingested per arbitrary unit (AU) of protease activity. The nutritional composition of the present invention was found to reduce the loss of endogenous protein, such as the loss of endogenously formed proteases. This was in particular the case for trypsin and/or chymotrypsin. Trypsin and chymotrypsin are in an infant the most important digestive proteases, since gastric pepsin is less active due to a higher pH in the stomach. In addition, proteases have been found to stimulate the endogenous loss of other proteins, such as mucins.

The nutritional composition of the present invention was found to reduce the protein load entering the colon.

These effects are observed compared to the situation before administration of the nutritional composition and/or to the situation compared to the administering of a standard nutritional composition not comprising the protein comprising fermented composition. It was found that these effects observed also come closer to the effects occurring in human milk fed infants compared to standard infant formula fed infants, since it was found that the proteolytic activity in faecal samples of exclusively breast fed human infants was lower than that of infants fed with a standard formula.

By these effects mentioned above the nutritional composition of the present invention improves gut health. In one embodiment the present nutritional composition for infants is for use in promoting gut health. Preferably the gut health is selected from the group consisting of improved well balanced intestinal microbiota, increased intestinal barrier function, decreased abdominal pain, prevention and/or treatment of intestinal inflammation, prevention and/or treatment of intestinal infections, prevention and/or treatment of diarrhea, prevention and/or treatment of colics or cramps, prevention and/or treatment of abdominal bloating, prevention and/or treatment of abdominal distention, and prevention and/or treatment of diaper rashes. In one embodiment the nutritional composition of the present invention improves gut health by prevention and/or treatment of constipation.

In particular the reduced amount of protein entering the colon will result in a more saccharolytic and less proteolytic activity of the intestinal microbiota. Fermentation of sugars instead of amino acids will result in a lower pH of the colon and/or in a reduced formation of toxic metabolites such as indols, phenols and amines. This also will result in more Bifidobacteria and/or Lactobacilli and/or less pathogenic bacteria in the intestinal microbiota. The amounts of bacteria can be expressed as cfu per g faeces and/or as a percentage based on cfu of total bacteria. Such improved intestinal microbiota will result in reduced intestinal infections and/or reduction in diarrhea.

In particular the reduced amount of proteases, more specifically serine proteases such as trypsin and chymotrypsin, will result in a reduced cleavage of PAR-2 with effect of an increased intestinal barrier function. An increased barrier function will result in reduced translocation of toxins, allergens and pathogens and hence in an advantageous effect on infection, diarrhea and/or inflammation. Also reduced PAR-2 cleavage will result in less abdominal pain perception Increased faecal proteolytic activity is in particular associated with IBD (inflammatory bowel disease) such as ulcerative colitis and with diarrhoegenic irritable bowel syndrome (IBS-D). Increased faecal proteolytic activity is in particular associated with the occurrence of diaper rashes. Therefore the present nutritional composition of the invention is preferably for use in treatment and/or prevention of diaper rashes. Furthermore, lower synthesis of proteases, concomitant with increase protein digestibility results in less energy and protein loss, which improves growth and development.

Colonic fermentation of proteins will result in changes of quality or quantity of gas formation and hence an increased abdominal bloating and/or abdominal distention. Hence the present nutritional composition will have an effect in reduction of abdominal bloating and/or abdominal distention and of disorders resulting thereof. Activation of protease activated receptors increases pain perception and has a negative effect on gut barrier function. Lower proteolytic activity may thus contribute to the increased prevention and/or treatment of colics or cramps.

All these above observed and mentioned effects are particularly important in young human subjects, since they need to grow and develop, have a more immature intestinal barrier and a less developed intestinal microbiota. In other words, in young human subjects, improving protein digestive effort, regulation of the endogenous protein release, restriction of protein loss, and decrease of protein load in the colon is most important. Hence, the nutritional composition is preferably used for feeding a human infant.

In one embodiment the invention concerns a method, preferably a non-therapeutic method, for

a reducing digestive effort,
b improving protein digestion efficacy
c reducing endogenous protein loss
d reducing endogenous secretion of proteases, and/or
e reducing the protein load entering the colon

compared to a nutritional composition not comprising a protein comprising composition fermented by lactic acid bacteria, said method comprising administering the nutritional composition for infants according to the present invention. Preferably for this method the nutritional composition for infants comprises lactose.

Preferably the present nutritional composition is used for providing nutrition to a human infant with an age of 36 month or below, more preferably of 18 month of age or below, even more preferably an infant with an age of 12 months of age or below, most preferably an infant with an age of 6 months or below. The present nutritional composition is preferably is enterally administered, more preferably orally.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one. Wt. means weight.

*Example 1:*

A double-blind, placebo-controlled, randomised, prospective study with parallel group design was conducted in healthy term born infants until the infants' age of one year. The test groups were:

Group 1: Infants on full formula feeding after 2 months at the latest receiving a standard non-hydrolysed cow's milk based formula (N=41). Group 2: Infants on full breastfeeding for at least 4 months (N=43).

Stool samples were collected at week 16 after birth and stored at -20°C until further analysis. Faecal proteolytic activity was measured with the EnzCheck protease fluorescence based assay kit (E6638, Invitrogen, Carlsbad, Ca, USA) for detecting metallo-, serine and sulfhydryl proteases. Faecal samples were 1000x diluted in 1x PBS, centrifuged at 13.000 rpm for 3 minutes to remove large particles, and 100 $\mu$l supernatant was added to 100 $\mu$l working BODIPY casein (10 $\mu$g BODIPY casein). Increase in fluorescence was measured at 25°C for a period of 10 minutes. Porcine pancreatin (Sigma, P1750) was used to prepare a calibration curve. To determine the origin of protease activity, the specific serine protease inhibitor AEBSF (Sigma, A8456) was added in each analysis with a final concentration of 5mM. Total protein in the faecal supernatants was determined using the BCA method (Pierce). Faecal proteolytic activity was expressed as arbitrary units (AU) based on pancreatin activity in USP, standard unit for activity according to United States Pharmacopeia) per gram faecal protein content. Statistics was performed with the Mann-Whitney U test. In the breast milk fed group the faecal proteolytic activity was 313 AU per mg protein (SEM 21) and in the group consuming standard infant formula the proteolytic acitivity was 406 AU per mg protein (SEM 33). The p-value was below 0.05.

The proteolytic activity was mainly derived from serine proteases (i.e. pancreatic enzymes or produced by colonic microbiota), since the specific serine protease inhibitor AEBSF blocked proteolytic activity with more than 70%.

*Example 2: Animal experiments*

To investigate protein digestion *in vivo*, pigs are first choice since they highly resemble human digestive physiology (Miller et al. 1987, Annu Rev Nutr, 7:361-82) and generate comparable true ileal nitrogen and amino acid (AA) digestibilities (Deglaire et al. 2009, Br J Nutr, 102(12):1752-9, Rowan et al. 1994, Br J Nutr, 71(1):29-42). In addition, a study by Moughan *et al.* has shown that three-week-old piglets can be used as a model for 6-month-old infants (Moughan et al. 1991, J Nutr, 121(10):1570-4). Therefore, AA digestibility of a fermented and standard infant formula (IF) was tested in a piglet model. A hydrolyzed formula (Nutrilon Pepti) was used to correct for endogenous AA losses according to the peptide alimentation method (Rutherfurd et al. 1998, J Dairy Sci, 81(4):909-17).

Six age- and weight matched male piglets (average weight 4.9 kg) were housed in groups from 2 weeks of age. At three weeks of age they received a T-cannula at the distal part of the terminal ileum after overnight fasting and were housed individually thereafter. From day 31 onwards the pigs received the following diets:

Diet 1: Lactofidus-1, a commercially available Infant Milk Formula marketed under brand name Gallia, comprising 100 % of a fermented milk-derived composition. Lactofidus is produced by fermenting with *B. breve* and *S. thermophilus*. It comprises 12.2 g protein per 100 g dry weight of which 60 wt.% casein and 40 wt.% whey protein. The pH is 5.6.

Diet 2: Nutrilon-2, a standard non-fermented follow on formula marketed under the brand name Nutricia, comprising non-hydrolysed protein, 9.3 g per 100 g dry weight of which 50 wt.% non-hydrolysed whey protein and 50 wt.% casein. The pH is 6.8.

Diet 3: Pepti-2, an IMF marketed under the brand name of Nutricia comprising 11.2 g protein per 100 g dry weight in the form of 100 wt.% extensively hydrolysed whey protein. The pH is 6.4. The fermented, standard and hydrolyzed powdered IMF was diluted in demineralised water (37°C) to a final dry matter content of 21.0%, 22.05% and 21.45%, respectively (the protein content being 2.46, 2.05, and 2.55 g/100ml). Chromium oxide was added to the IMF powder as indigestible marker. Until day 31, the piglets received a 1:1:1 w/w/w mixture of diet 1, 2 and 3. From day 31 to day 36 the piglets received either diet 1, 2 or 3 in a Latin Square design according to the feeding scheme of Table 1. Pigs were fed at 7:00 h, 9:30 h, 14:00 h and 16:00 h. Ileal digesta were collected at day 32, 34 and 36 from 8.00 h till 17.00 h, via the T cannula in small bags which were stored on ice immediately when filled. All digesta samples were weighed, pH measured and stored at -20°C until further processing. For enzymatic activity measurements a subsample (2 ml) was centrifuged (13.000 g for 10 minutes at 4°C) and supernatants were aliquoted and stored at -80°C.

Table 1: Feeding scheme

| Piglet | Day 31-32 | Day 33-34 | Day 35-36 |
|--------|-----------|-----------|-----------|
| 1 | 1 | 2 | 3 |
| 2 | 1 | 3 | 2 |
| 3 | 2 | 1 | 3 |
| 4 | 2 | 3 | 1 |
| 5 | 3 | 1 | 2 |
| 6 | 3 | 2 | 1 |

Dry matter (DM) (gravimetry at 80°C), chromium oxide (Cr) (inductively coupled plasma mass spectrometry), crude protein (CP) (Kjeldahl method, N x 6.25) and amino acid (AA) composition (HPLC after 6M HCL hydrolysis) were analyzed in freeze-dried digesta samples and diet powders.

Total proteolytic activity in the ileal digesta was determined using the EnzCheck protease fluorescence based assay kit (E6638, Invitrogen, Carlsbad, Ca, USA) for detecting metallo-, serine and sulfhydryl proteases. Digesta were diluted 750x with 10 mM Tris-HCl, pH 7.8, and 100 $\mu$l samples were added to 100 $\mu$l working BODIPY casein (10 $\mu$g BODIPY casein). Increase in fluorescence was measured at 25°C for a period of 10 minutes. Porcine pancreatin (Sigma, P1750) was used to prepare a calibration curve. To determine the origin of protease activity, the specific serine protease inhibitor AEBSF (Sigma, A8456) was added in each analysis with a final concentration of 5 mM. Activity is expressed as arbitrary unit (AU) (based on pancreatin activity in USP).

Trypsin activity was measured by using $N_\alpha$-Benzoyl-L-Arginne Ethyl Ester (BAEE, Sigma B4500) as substrate and measuring the absorbance change at 25°C 253 nm (according to the manufacturer's instructions). Bovine trypsin (Sigma, T9201) was used to prepare a calibration curve. Activity is expressed as arbitrary unit (AU) (based on trypsin activity in U).

Chymotrypsin was measured by using $N_\alpha$-Benzoyl-L-Tyrosine Ethyl Ester (BTEE, Sigma B6125) as substrate and measuring the absorbance change at 25°C at 256 nm. Bovine chymotrypsin (Sigma, C3142) was used to prepare a calibration curve. Activity is expressed as arbitrary unit (AU) (based on chymotrypsin activity in U).

Equations used to calculate AA digestibility (units are in $\mu$g/g DMI):

$$\text{Ileal AA flow} = \text{ileal AA} \times \frac{\text{Cr diet}}{\text{Cr digesta}}$$

$$\text{Endogenous AA flow} = \text{ileal AA (MW>10 kD)} \times \frac{\text{Cr diet}}{\text{Cr digesta}}$$

$$\text{Apparent ileal AA digestibility (\%)} = \frac{\text{AA intake} - \text{ileal AA flow}}{\text{AA intake}} \times 100\%$$

$$\text{True ileal AA digestibility (\%)} = \frac{\text{AA intake} - (\text{ileal AA flow} - \text{endogenous AA flow})}{\text{AA intake}} \times 100\%$$

The results were analyzed using Univariate Analysis of Variance (GLM procedure). Differences between diets were considered significant with $p<0.05$ according to the LSD test.

Table 3. Diet intake, ileal digesta characteristics & ileal AA digestibility

|  | Nutrilon | Lactofidus | Pepti | Pooled SE |
|---|----------|------------|-------|-----------|
| DM intake (g/day) | 403[a] | 388[b] | 391[b] | 3.641 |
| CP intake (g/day) | 37.4[a] | 45.4[b] | 46.4[c] | 0.317 |
| Total AA intake (g/day) | 39.1[a] | 47.7[b] | 50.2[c] | 0.347 |
| Ileal digesta pH | 7.91 | 7.95 | 7.90 | 0.06 |

(continued)

| | Nutrilon | Lactofidus | Pepti | Pooled SE |
|---|---|---|---|---|
| Ileal digesta CP (g/g CPI) | 0.16[a] | 0.08[b] | 0.16[a] | 0.01 |
| Total ileal digesta CP (g/day) | 5.96[a] | 3.64[b] | 7.27[c] | 0.279 |
| Apparent ileal AA digestibility (%) | 89.1[a] | 94.4[b] | x | 0.53 |
| True ileal AA digestibility (%) | 97.1[a] | 100.8[b] | x | 0.61 |

NS = not significant, x = not determined.
[a-c] Values with different letters within the same row are different ($p < 0.05$)

Table 4. Ileal proteolytic enzyme activities

| | Nutrilon | Lactofidus | Pepti | Pooled SE |
|---|---|---|---|---|
| Total proteolytic activity | | | | |
| AU x $10^3$/8h | 1599[a] | 477[b] | 759[b] | 140 |
| AU x $10^3$/g CPI | 41.4[a] | 10.5[b] | 16.5[b] | 3.3 |
| Trypsin activity | | | | |
| AU x $10^3$/8h | 908[a] | 334[b] | 621[c] | 74 |
| AU/g CPI | 23.9[a] | 7.4[b] | 13.5[c] | 1.6 |
| Chymotrypsin activity | | | | |
| AU/ 8h | 665[a] | 294[b] | 486[ab] | 73 |
| AU/g CPI | 17.2[a] | 6.5[b] | 10.3[b] | 1.8 |

NS = not significant, x = not determined.
[a-c] Values with different letters within the same row are different ($p < 0.05$)

The results in Table 3 show that the pH of the ileal digesta was the same. The amount of crude protein intake was highest in the hydrolysed formula and in the fermented formula. Interestingly, the amount of total protein in the ileal digesta and the relative amount of protein based on protein intake was significantly lower in the piglets consuming fermented formula than in the other two groups. This flow of protein to the colon is therefore lowest in piglets consuming fermented formula. The apparent and true ileal amino acid digestibility was higher in piglets consuming the fermented formula than in the group consuming the standard formula.

The results in Table 4 show that the amount of proteolytic activity secreted during the 8 h of collection is lowest in piglets consuming Lactofidus, the fermented infant formula, when compared with standard non fermented formula or a hydrolysed, predigested IMF. This is the case for total proteolytic activity as well as for trypsin and chymotrypsin. This effect is also observed when based on amount of protein intake. The proteolytic activity is mainly derived from serine proteases (i.e. pancreatic enzymes such as trypsin and chymotrypsin), since the specific serine protease inhibitor AEBSF blocked proteolytic activity with more than 90%. So, the amount of proteolytic activity entering the colon is lower upon consumption of a fermented formula, which makes it more similar to the situation in breast fed infants (see example 1). Surprisingly the apparent and true protein digestibility is the highest when consuming the fermented formula, even though the lowest amount of proteases is formed. This means that the digestive efficacy (in [Protein ingested/protease activity in AU) is higher. Likewise, the amount of protealytic activity per g protein intake is lower, which is indicative of a decreased digestive effort.

*Example 3: Low protein infant formula*

A reconstituted infant formula comprising per 100 ml:

    12.3 g dry matter, 66 kcal
    1.19 g protein (bovine whey protein/casein in 1/1 weight ratio)
    7.76 g carbohydrate (mainly lactose)
    3.36 g fat (mainly vegetable fat).

Of this composition 30 % based on dry weight is derived from lactofidus-1 as described in example 2. The composition further comprises, vitamins, minerals, trace elements and other micronutrients according to international directives.

**Claims**

1. A nutritional compostion for infants comprising:

   a. protein

      i. in an amount of from 5 to 20 wt.% based on dry weight of the nutritional composition, and
      ii. in an amount of 6% to 7.4% calories based on total calories of the nutritional composition,

   b. lipids in an amount of 35 to 60 % calories based on total calories of the nutritional composition, and
   c. carbohydrates in an amount of 40 to 60 % calories based on total calories of the nutritional composition,

   wherein the nutritional composition comprises at least 10 wt.% of a protein comprising composition fermented by lactic acid bacteria based on dry weight of the nutritional composition.

2. The nutritional composition according to claim 1, wherein the nutritional composition comprises from 0.10 to 1.5 wt. % of the sum of lactate and lactic acid based on dry weight and wherein the sum of L-lactic acid and L-lactate is more than 50 wt. % based on the sum of total lactic acid and lactate.

3. The nutritional composition according to any one of the preceding claims wherein the protein comprising composition fermented by lactic acid bacteria, is fermented by *Streptococcus thermophilus*, and/or *Bifidobacterium breve.*

4. The nutritional composition according to any one of the preceding claims wherein the lactic acid bacteria are inactivated after fermentation.

5. The nutritional composition according to any one of the preceding claims which is a liquid having a pH from 5.5 to 6.0.

6. The nutritional composition according to anyone of the preceding claims wherein the nutritional composition is selected from the group consisting of an infant formula, a follow on formula, a toddler formula and a growing up milk, preferably infant formula.

7. The nutritional composition according to any one of the preceding claims, wherein the protein comprises whey protein and casein in a weight ratio of 20:80 to 80:20, more preferably 35:65 to 55:45.

8. Nutritional composition according to any one of the preceding claims for use in promoting gut health.

9. The nutritional composition for use according to claim 8 wherein the gut health is selected from the group consisting of improved well balanced intestinal microbiota, increased intestinal barrier function, decreased abdominal pain, prevention and/or treatment of intestinal inflammation, prevention and/or treatment of intestinal infections, prevention and/or treatment of diarrhea, prevention and/or treatment of colics or cramps, prevention and/or treatment of abdominal bloating, prevention and/or treatment of abdominal distention, and prevention and/or treatment of diaper rashes.

10. The nutritional composition for use according to claim 8 or 9, in an infant with an age of 0 to 36 months, preferably 0 to 12 months.

11. A non-therapeutic method for

    a reducing digestive effort,
    b improving protein digestion efficacy
    c reducing endogenous protein loss
    d reducing endogenous secretion of proteases, and/or
    e reducing the protein load entering the colon

compared to a nutritional composition not comprising a protein comprising composition fermented by lactic acid bacteria, said method comprising administering a nutritional composition of any one of the preceding claims.

12. The method according to claim 11, wherein the composition is administered to an infant with an age of 0 to 36 months, preferably 0 to 12 months.

# EP 2 520 181 A1

Europäisches
Patentamt
European
Patent Office
Office européen
des brevets

## EUROPEAN SEARCH REPORT

Application Number

EP 11 16 4461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/151330 A1 (NUTRICIA NV [NL]; SCHMITT JOACHIM [DE]; PERRIN EMMANUEL [FR]; STAHL BE) 17 December 2009 (2009-12-17) | 1-10 | INV.<br>A23L1/30<br>A61P1/00<br>A23L1/29 |
| Y | * page 21, line 1 - line 18; claim 6; examples 5,6 *<br>* page 23, line 1 - page 25, line 8 *<br>----- | 11,12 | A23L1/305<br>A61K35/74 |
| A | WO 2010/069737 A1 (NESTEC SA [CH]; ARIGONI FABRIZIO [CH]; BRUESSOW HARALD [CH]; CAVADINI) 24 June 2010 (2010-06-24)<br>* page 5, line 6 - line 30; example 1 *<br>----- | 1-12 | |
| X | THIBAULT H ET AL: "EFFECTS OF LONG-TERM CONSUMPTION OF A FERMENTED INFANT FORMULA (WITH BIFIDOBACTERIUM BREVE C50 AND STREPTOCOCCUS THERMOPHILUS 065) ON ACUTE DIARRHEA IN HEALTHY INFANTS", JOURNAL OF PEDIATRIC GASTROENTEROLOGY AND NUTRITION, RAVEN PRESS, NEW YORK, NY, US, vol. 39, no. 2, 1 August 2004 (2004-08-01), pages 147-152, XP009043383, ISSN: 0277-2116, DOI: DOI:10.1097/00005176-200408000-00004 | 1-10 | |
| Y | * page 147, right-hand column, last paragraph - page 148, left-hand column, paragraph 5 *<br>* abstract *<br>----- | 11,12 | TECHNICAL FIELDS SEARCHED (IPC)<br><br>A23L<br>A61K |
| A | EP 1 841 330 A1 (NESTEC SA [CH]) 10 October 2007 (2007-10-10)<br>* paragraph [0020] - paragraph [0036]; claims 2,3,4,10 *<br>-----<br><br>-/-- | 1-12 | |

The present search report has been drawn up for all claims

| Place of search | Date of completion of the search | Examiner |
|---|---|---|
| Munich | 14 July 2011 | Munteanu, Ioana S. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 11 16 4461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/070613 A2 (UNIV LONDON [GB]; SINGHAL ATUL [GB]; LUCAS ALAN [GB]) 24 June 2010 (2010-06-24) * paragraph [0057] - paragraph [0058]; claims 1,2 * ----- | 1-12 | |
| Y | EP 1 020 123 A1 (SITIA YOMO SPA [IT]) 19 July 2000 (2000-07-19) | 11,12 | |
| A | * paragraph [0009] * ----- | 1-10 | |
| Y | US 2007/160589 A1 (MATTSON PETER H [US]) 12 July 2007 (2007-07-12) | 11,12 | |
| A | * paragraph [0003] * ----- | 1-10 | |
| Y | EP 1 145 643 A1 (CONNOLLY PHILLIP [US]) 17 October 2001 (2001-10-17) | 11,12 | |
| A | * paragraph [0026] * ----- | 1-10 | |

TECHNICAL FIELDS
SEARCHED        (IPC)

The present search report has been drawn up for all claims

| Place of search | Date of completion of the search | Examiner |
|---|---|---|
| Munich | 14 July 2011 | Munteanu, Ioana S. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                     EP 11 16 4461

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2011

| Patent document cited in search report | | Publication date | Patent family member(s) | | Publication date |
|---|---|---|---|---|---|
| WO 2009151330 | A1 | 17-12-2009 | AR | 072141 A1 | 11-08-2010 |
| | | | AR | 072142 A1 | 11-08-2010 |
| | | | CN | 102065867 A | 18-05-2011 |
| | | | CN | 102118976 A | 06-07-2011 |
| | | | EP | 2285387 A1 | 23-02-2011 |
| | | | EP | 2293677 A1 | 16-03-2011 |
| | | | EP | 2293803 A1 | 16-03-2011 |
| | | | WO | 2009151329 A1 | 17-12-2009 |
| | | | WO | 2009151331 A1 | 17-12-2009 |
| | | | US | 2011117077 A1 | 19-05-2011 |
| | | | US | 2011097437 A1 | 28-04-2011 |
| WO 2010069737 | A1 | 24-06-2010 | NONE | | |
| EP 1841330 | A1 | 10-10-2007 | AR | 052074 A1 | 28-02-2007 |
| | | | AT | 498324 T | 15-03-2011 |
| | | | AU | 2005321326 A1 | 06-07-2006 |
| | | | BR | PI0519507 A2 | 10-03-2009 |
| | | | CA | 2593033 A1 | 06-07-2006 |
| | | | CN | 101087539 A | 12-12-2007 |
| | | | WO | 2006069918 A1 | 06-07-2006 |
| | | | ES | 2359573 T3 | 24-05-2011 |
| | | | PT | 1841330 E | 14-03-2011 |
| | | | US | 2010022451 A1 | 28-01-2010 |
| | | | US | 2011028389 A1 | 03-02-2011 |
| | | | ZA | 200706201 A | 29-10-2008 |
| WO 2010070613 | A2 | 24-06-2010 | NONE | | |
| EP 1020123 | A1 | 19-07-2000 | BR | 9906141 A | 06-02-2001 |
| | | | MX | PA00000628 A | 22-07-2002 |
| US 2007160589 | A1 | 12-07-2007 | NONE | | |
| EP 1145643 | A1 | 17-10-2001 | NONE | | |

EPO FORM P0459

For more details about this annex : see Official Journal of the European Patent Office, No. 12/82

**EP 2 520 181 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 778885 A

- FR 2723960

### Non-patent literature cited in the description

- **ALM.** *J Dairy Sci,* 1982, vol. 65, 509-514
- **VASS et al.** *Acta Medica Hungarica,* 1984, vol. 41, 15-161
- *Compagnie Gervais Danone at the Collection Nationale de Cultures de Microorganismes (CNCM,* 23 August 1995
- **MILLER et al.** *Annu Rev Nutr,* 1987, vol. 7, 361-82

- **DEGLAIRE et al.** *Br J Nutr,* 2009, vol. 102 (12), 1752-9
- **ROWAN et al.** *Br J Nutr,* 1994, vol. 71 (1), 29-42
- **MOUGHAN et al.** *J Nutr,* 1991, vol. 121 (10), 1570-4
- **RUTHERFURD et al.** *J Dairy Sci,* 1998, vol. 81 (4), 909-17